# EUROPEAN PATENT SPECIFICATION

(11) **EP 2 220 474 B1**
(45) Date of publication and mention of the grant of the patent: **22.07.2015**
(21) Application number: 07866800.1
(22) Date of filing: 13.12.2007
(51) Int. Cl.: G01N 1/14, A61B 10/00

(54) **METHOD OF COLLECTION AND PRESERVATION OF FLUIDS AND/OR MATERIALS, IN PARTICULAR OF ORGANIC FLUIDS AND/OR MATERIALS CONTAINING STEM CELLS, AND DEVICE EMPLOYABLE IN SUCH METHOD**
VERFAHREN ZUM SAMMELN UND KONSERVIEREN VON FLÜSSIGKEITEN UND/ODER MATERIALIEN, INSBESONDERE VON ORGANISCHEN FLÜSSIGKEITEN UND/ODER MATERIALIEN MIT STAMMZELLEN, SOWIE BEI EINEM SOLCHEN VERFAHREN EINSETZBARE VORRICHTUNG
PROCÉDÉ DE COLLECTE ET DE CONSERVATION DE FLUIDES ET/OU DE MATIÈRES, EN PARTICULIER DE FLUIDES ORGANIQUES ET/OU DE MATIÈRES CONTENANT DES CELLULES SOUCHES, ET DISPOSITIF UTILISABLE DANS UN TEL PROCÉDÉ

(43) Date of publication of application: 25.08.2010
(73) Proprietor: Biocell Center S.p.a., 21052 Busto Arsizio (IT)
(72) Inventor: MAGGI, Federico, I-28010 Colazza (IT); REGUZZONI, Marco, Giovanni, I-21052 Busto Arsizio (IT); SIMONI, Giuseppe, I-27100 Pavia (IT); MACCAGNAN, Simone, I-21040 Castronno (IT)
(74) Representative: Tarabbia, Luigi
(86) International application number: PCT/IT2007/000868
(87) International publication number: WO 2009/075001

(56) References cited:
- EP-A- 1 554 931
- WO-A-90/13261
- WO-A-03/066585
- WO-A-2005/099599
- US-A- 5 000 192
- US-A- 5 103 836
- US-A- 5 465 768
- US-A- 5 714 696
- US-A- 5 776 781
- US-A- 5 811 696
- US-A1- 2007 020 225
- US-B1- 6 291 178
- US-B1- 6 360 620

## Description

The present invention relates to a method of collection and preservation of fluids and/or materials containing stem cells, such method being employable in different operating fields, such as sampling and preservation of organic fluids in the clinical/laboratorial sphere.

It is known that in different production fields sampling of a fluid substance from a given closed volume is made necessary, as it may happens in prenatal medical examples, and also as far as working of some electronic printed circuits is concerned.

In the first one of the two application fields mentioned above by way of example, while tests such as amniocentesis and CVS (i.e. Chorionic Villi Sampling) are being executed, a first step is in particular contemplated in which a first sampling of amniotic liquid (about 3 cc) is carried out by means of a suitably positioned syringe so as to perforate the pregnant woman's abdominal wall and placenta in the less invasive manner as possible; subsequently, while the needle is maintained implanted during the first sampling, the syringe body is changed in order to obtain a second more voluminous sampling (about 10 cc of the amniotic liquid).

Generally, the liquid taken during the first sampling is discarded, essentially due to the fact that insertion of the needle through the abdominal wall and placenta gives rise to creation of a substantially cylindrical residue of the mother's organic tissue (that in the particular field is usually referred to as "frustule" or "fragment"), which obviously interferes with the quality of the taken sample of fluid and/or material.

It is however to be pointed out that presently the known art does not contemplate any method involving long-term use of the amniotic liquid or chorionic villi, above all in connection with the possibility of preserving, taking out and subsequently manipulating the stem cells that can be found suspended therein.

The second application field mentioned above, on the contrary, involves a particular method of printing electronic circuits in which a particular gel is used that covers the board under working (which in turn is "mapped" by means of a laser beam or the like); in this method, the physico-chemical features of the gel adversely affect the conductivity of the electronic circuits printed by means of the laser beam (passing through the gel itself), and it is therefore essential that samples of the gel be carried out periodically in order to check composition of same.

In the application examples briefly described above (but also in many other operating fields) it is therefore necessary to operate on a given substance than can be in the fluid state and is adapted to be analyzed/examined; at the same time, this substance is to be maintained in an operating volume that must be as much as possible isolated from external agents (atmosphere, non-sterile environments, foreign substances and so on).

The just described known art, despite being rather widely spread, it has some drawbacks.

First, execution of known methods for sampling of fluids and/or materials (of organic or non-organic nature) is highly exposed to risks of contamination of the taken samples, with all negative consequences resulting therefrom.

In addition, referring in particular to organic fluids and/or materials containing stem cells, it is to be pointed out that implementation of a method enabling an efficient and accurate collection and preservation of same is hitherto unknown, neither known is a method capable of ensuring the necessary hermetic sealing and sterility conditions (or in any case the conditions of maximum separation from undesirable environmental agents of macro-or microscopic nature) for putting into practice the "working" and manipulation techniques that can (or could in the future) be applied to the stem cells themselves.

Furthermore, with use of common syringes the collected liquid is required to be poured from the true syringe into a suitable container for storage and/or subsequent analysis; however this intermediate transfer can expose the collected sample to an undesirable contact with the surrounding environment that can lead to contamination and/or mistakes in the analysis.

This drawback is particularly serious in the clinical field, since the possibility of breaking isolation between the fluid collection volume and the surrounding environment can cause entry of pathogenic agents into the sampled fluid, but above all into the patient's body; in the case of prenatal tests, the consequences can be doubly adverse, since obviously possible pathologies can arise both for the pregnant woman and the unborn fetus.

On the other hand, should long-term preservation of the taken fluid be necessary, and in particular should addition of suitable preserving substances to the fluid be required, once more the syringes of the conventional type are not able to carry out this operation without exposing the taken ample to the external environment (which involves exposing the fluid to contamination risks). US 5000192, WO 90/13261 shows examples of sampling devices for amniotic fluid.

In the light of the above, the present invention aims at conceiving a process enabling well precise volumes of fluid and/or material to be sampled in a fully safe manner (as regards external contamination) and more quickly than with traditional methods.

Still more generally, the present invention aims at conceiving a method capable of sampling and preserving organic fluids (such as the amniotic fluid for example, and/or the material obtained when a CVS procedure is executed) containing stem cells, so that it is possible to work on the stem cells therein contained and maintained in a preservation state, even after an indefinite period of storage.

Still as regards the method, the present invention wishes to make available a process enabling possible admixing of preserving substances to the sampled fluids and/or material, in a short period of time and with the greatest reliability and accuracy (obviously while fully observing the requirement of isolating them from the external environment also during the step of adding the preservative).

The foregoing and further aims are achieved by a method of collection and preservation of fluids and/or materials, in particular organic fluids and/or materials containing stem cells, as well as by a device employable in such a method, in accordance with the present invention, having the features shown in the appended claims and hereinafter illustrated in an embodiment thereof given by way of non-limiting example.

From the operating point of view, the present invention therefore contemplates implementation of a method of collection and preservation of a fluid and/or a material (that can be of organic nature, as in the embodiment of the method described later on, and that preferably will contain a certain amount of stem cells), which mainly (but not exclusively) comprises the following steps:
- first of all, a sampling volume is determined, that preferably will be closed and/or separated from the surrounding environment (such as a pregnant woman's placenta, or the inside of a bottle of wine under maturation and so on);
- once determined the sampling volume on which to operate, a predetermined amount of fluid and/or material is taken from this sampling volume; and
- this predetermined amount of fluid and/or material is confined into a collection volume.

Advantageously, the determination of the amount of fluid and/or material and the confining of such amount in the sampling volume are carried out simultaneously, and in addition these two operating steps are contemporaneous with a step of maintaining the hydraulic and/or pneumatic and/or microbiological isolation between the sampling volume and the collection volume.

At this point, it will be appreciated that a difference exists between the method of the invention and the method of the known art; in fact, while in known methods there is always a moment at which the sampled fluid and/or material actually comes into contact with the environment external to the collection volume (let us think of the moment of change of needle in the amniocentesis procedures carried out in a traditional manner or in any case the moment at which the sampling probe is extracted from the collection volume in other, fields), according to the present process, not only a perfect separation is ensured at any time between the sampled fluid and/or material and the fluid and/or material still remaining in the sampling volume, but at the same time a permanent and continuous separation is ensured over time between the external environment and the amount of sampled fluid and/or material, both during the true sampling and during the subsequent operating steps involving the sampled fluid and/or material.

The method according to the invention is particularly appreciable in the clinical field, where for instance it is possible to operate on the amniotic liquid or also, if necessary, on an organic sample comprising a predetermined amount of chorionic villi; this method can further be used both in traditional amniocentesis and/or CVS operations and in a more complex procedure for extraction and preservation of the amniotic liquid (that in turn is correlated with a preservation process and possible subsequent re-use of the stem cells contained inside the amniotic liquid and/or the chorionic villi).

In the last-mentioned particular application, determination of the sampling volume comprises a sub-step of controlling the fetus' position and/or the placenta's position; in addition, still to the aims of a correct execution of the process (and for reasons connected with control of the health both of the pregnant woman and the fetus), a step is provided for control of a predetermined number of physico-biological parameters of the fetus and/or the pregnant woman.

As regards operation, it is therefore possible to contemplate a step of taking an additional sample of fluid and/or material.

To the aims of the present invention, it is important to emphasize that maintenance of the hermetic sealing and/or isolation between the sampling volume and the external environment is ensured, due to the presence of suitable isolation means automatically and instantaneously starting operation at the separation instant.

The method hereabove described can be integrated with a step of storing and preserving the fluid and/or material; this operating step can conveniently comprise a sub-step of mixing the fluid and/or material with at least one preserving agent (for example a dimethyl sulfoxide-based preservative, or DMSO as commonly termed in the chemical field).

In accordance with the present invention, the mixing sub-step too takes place while ensuring the full separation and hermetic sealing of the collected fluid and/or material relative to the surrounding environment.

Conveniently, the step of preserving the sampled fluid and/or material further comprises a sub-step of cooling the fluid and/or material under a predetermined preservation temperature; this cooling sub-step can precede the sub-step of spraying and/or mixing the fluid and/or material with at least one preserving agent.

After the desired amount of fluid and/or material has been taken, and after possibly doing the necessary to enable preservation of same for an indefinite period of time, it is also possible to transfer the fluid and/or material into suitable storage means.

This step of transferring the fluid and/or material into storage means can be conveniently optimized from the logistic point of view by allocating a series of data at least concerning positioning (but also, depending on specific requirements, data relating to other parameters, such as sampling data, environmental conditions at the sampling moment, operator who has carried out sampling, and so on) to each fluid and/or material sample; subsequently, these data can be stored into suitable storage systems.

As regards execution, the present method can advantageously apply for collection and preservation (as well as for a possible and subsequent new processing or culturing) of organic fluids and/or materials containing stem cells (such as the amniotic liquid and/or the chorionic villi that can be taken from a pregnant woman, for example).

It is to be noted in this connection that stem cells taken from the amniotic liquid can be used as a cellular therapy source for treatment of pathologies in humans: in order to ensure use of these cells it is of the greatest importance to ensure sterility of the cellular product with which the patient will be re-inoculated.

Operatively, sterility of the amniotic liquid sample (or the sample of chorionic villi) during all the manipulation steps following true sampling, or possibly also the previously described preservation/storage step, is ensured by a suitable laboratory instrument known in the technical field with the name of "isolator".

It is to be noted at this point that use of the isolator, constitutes an important novelty relative to the methodologies presently applied for manipulating stem cells: actually, operators presently acting on stem cells move (and manipulate) the biological material inside sterile rooms and therefore, although they have all possible protections (masks, gloves and others) at their disposal, they yet represent a contamination source for the organic fluid or material under processing, since they transfer (at least through breathing) an important charge of bacteria or in any case of polluting agents (powders and dust, residues resulting from exfoliation of the skin and so on) into the same environment where the stem cells are.

On the contrary, use of the isolator enables full separation between the operator (and above all any environmental alteration/pollution source connected with the operator's physical presence) and the fluid and/or material containing the stem cells, or even the stem cells already separated from the fluid/material together with which they have been taken from a given patient.

From a structural point of view, the isolator employable in the present method mainly comprises a work chamber which practically is a steel box completely isolated from the outside, provided with a filtering system having filters of the "Hepa" type and where access for the operator takes place through use of suitable gloves jutting out inside the box and sealingly connected with one of the box walls: conveniently, this box is pressurized to a greater pressure than the inlet chamber. Also present is an inlet chamber (also termed "pass-box") for introduction of the sample and the biological material to be processed, which chamber is provided with interlocking doors that do not allow direct communication between the work chamber and the surrounding environment.

Likewise, an outlet chamber is present which has a sterile sample-collecting bag: this outlet chamber is brought into communication with the work chamber by interlocking doors.

Finally, a sterilization system is present for processing the biological sample and sterilizing the outer surface of the biological-sample container; the sterilization process takes place by use of hydrogen peroxide (H₂O₂) that is introduced into the isolator before each work step and between processing of two different biological samples in order to ensure absolute sterility during the manipulation step; during this step the "particle count" and/or "microbiological count" parameters will be continuously analyzed.

On the contrary, as regards freezing of the stem cells, the following is done.

From about 20 ml of amniotic liquid obtained, through amniocentesis (or from a corresponding amount of chorionic villi, obtained through CVS - Chorionic Villi Sampling), an aliquot part of 2-2.5 ml will be taken, then the samples contained in 15 ml test tubes with conical bottom and screw plug are centrifuged at 2000 rpm for 10 minutes.

After centrifugation, the test tube is inserted into the isolator and without disturbing the so-called "cellular-pellet", the so-called "supernatant" is taken and preserved for preparing the freezing solution for the sample with final 10% DMSO.

This solution is cooled using a suitable cooling apparatus positioned inside the isolator and 1 ml thereof is used to suspend the amniocyte pellet again, said pellet being then inserted into a suitable test tube for freezing (which instead has been caused to come out of the isolator's outlet chamber and then frozen by a programmable freezer).

At the end of the freezing step, the sample is preserved in suitable storage containers containing liquid nitrogen.

Generally freezing is carried out on non-hematic samples and on samples non containing meconium that have' been taken 24-48 hours earlier.

Defrosting of the stem cells is carried out by taking the sample out of the liquid nitrogen, positioning it in ice and bringing it into a 37°C thermostat in the shortest period of time.

After about 3 minutes, the defrosted sample is transferred into the isolator and then drop-wise transferred into a 15 ml test tube with conical bottom and screw plug (this test tube contains about 9 ml of washing medium).

At the end of the addition, the test tube is caused to come out of the isolator's outlet chamber and is subsequently centrifuged at 1500 rpm for 10 minutes.

After centrifugation, the test tube is inserted into the isolator and without disturbing the cellular pellet, the supernatant is taken; at this point, the well-containing pellet is suspended again with a suitable cell-growth substance (termed "medium") in an amount of about. 2 ml; the cellular suspension will be transferred into a suitable flask (in jargon termed "T25") for expansion.

It is to be noted that in accordance with the present invention, the method of collection and preservation of organic fluids and/or materials containing stem cells (such as the amniotic liquid and/or Chorionic villi that can be taken from a pregnant woman) can therefore comprise a cryogenic preservation step and possibly a subsequent unstoring step that in turn comprises at least a defrosting sub-step.

More generally, it is also to be noted that the collection and preservation method applicable to organic fluids and/or materials containing stem cells can advantageous comprise a sampling step (carried out on a suitable "collection volume") that is executed while a perfect microbiological, atmospheric and physical isolation is continuously maintained between the sampling volume, the sample of collected fluid and/or material and the collection portion wherein the fluid/material is confined.

As regards the above mentioned isolator, it is to be noted that within the scope of the present invention the structure of the latter has been suitably conceived for maximizing the efficiency of the work method.

First of all, it will be appreciated that the size of the isolator's work chamber (which can be considered by way of example as a cubic volume having sides of about 80 cm) has been suitably studied so as to reduce the sizes of the inner surfaces and the volume: this geometric effect greatly reduces the time required for decontamination of the work chamber and also reduces consumption of sterilizing agents (such as hydrogen peroxide) required for decontamination.

The isolator also offers the possibility of pre-cooling the freezing solution by using a thermo-block (made of steel) housed inside the work chamber: this positioning of the thermo-block ensures maximum sterility for the biological sample and the work area, unlike known systems exploiting a more traditional cooling by ice (which is not sterile and cannot be sterilized) contained in a container that in turn is not sterile.

The structural features of the isolator in this manner offer the possibility of keeping the biological sample inside the work chamber at a controlled temperature (i.e. temperature-regulating means is present in the work chamber, so that suitable comfort conditions can be achieved both for the product and the operator).

At the same time the isolator is provided with sealed and/or hermetic and/or sterile packaging means located at the outlet chamber: this sealed and/or hermetic and/or sterile packaging means offers the possibility of packaging the product coming out of the isolator in an aseptic manner, by a suitable sterile "rolled bag" (which in turn is useful for the purpose of carrying the sample to the cryogenic freezing and/or cryogenic preservation point).

In addition, due to the presence of the sterile "rolled bag" or equivalent means, possible working waste can also be eliminated without contaminating any part of the isolator (and therefore avoiding further sterilization cycles being carried out).

The present isolator further has suitable filters (made of Gore-tex for example) that are operatively active on a line for admission of sterilizing agents, and preferably a line for admission of hydrogen peroxide; in this manner a further lowering of the decontamination time is obtained.

In order to reach an ergonomic improvement for the operator, the isolator also has suitable positioning means for a biological sample (or in other words, for the sampled fluid and/or material), as well as for the material required for processing: conveniently, this means can consist of a steel rack having the same surface finish degree as the isolator's walls.

The isolator can also be provided with self-governing movement means (a train of pivoting wheels or the like, for example) that allow displacement of same inside the room: practically, the self-governing movement means allows the isolator to be shifted to the desired points, thus facilitating cleaning of the room and maintenance of the isolator itself, for example (or in any case offering the possibility of shifting the isolator to points in the room or laboratory that are more advantageous from an operating point of view).

As already said, the isolator also comprises sterilization means acting at least on the work chamber and/or the inlet chamber and/or the outlet chamber: this means can conveniently atomize one or more sterilizing agents so as to decontaminate every point of the isolator itself.

For completion of the different operating aspects of the isolator, suitable sensor means is then present which acts at least in the work chamber (but, if necessary, also in the inlet chamber and/or the outlet chamber), said sensor means being able to measure:
- a microbiological sampling (typically, by an air intake positioned close to the work region, so as to have a truer and safer view of the working neighborhood;
- an air speed within the work chamber (these sensors can be possibly coupled with filtering means, comprising one or more filters of the "hepa" type for example, and/or with venting means acting on the work chamber and/or the inlet chamber and/or the outlet chamber);
- a given number of particle values of the air during the whole process;
- a possible residual amount of sterilizing agents still in the work chamber after a sterilization process.

Depending on the different occasions, the above described isolator can also be used separated from the method being the object of the present invention, i.e. in other industrial and/or laboratory processing methods on several different types of materials and/or (biological and non-biological) samples.

The invention enables achievement of important advantages.

In terms of operation, the present invention therefore enables accomplishment of a quicker collection, and preservation method as compared with traditional methods in use; this method also allows two operations that are generally carried out at different times (and therefore are time-consuming) to be integrated into a single operating step.

It will be appreciated that this method is in any case compatible with the already known operating methodologies and that a different degree of preparation by the staff putting it into practice is not required.

Finally, the present invention enables low production and sale costs to be achieved both in terms of manufacture of the collection and preservation device and in terms of cheap management of the sampling/storage/analysis works that are necessary in a great number of technological fields.

A method of collection and preservation of organic fluids and/or materials containing stem cells comprises the following steps: taking fluid/material containing stem cells from a sampling volume and confining it in a sample and preserving the sample; the step of taking fluid and/or material is performed while contiguously maintaining a perfect microbiological, atmospheric and physical isolation between the sampling volume and the collection volume and the method further comprises a step of manipulating the sample under sterility conditions in an isolator apparatus comprising a pressurized and isolated work chamber with gloves jutting out at the inside of the box, an inlet chamber for introduction of the sample and provided with interlocking doors not allowing direct communication between the work chamber and the environment, an outlet chamber connected with the work chamber brought into communication with the work chamber by means of interlocking doors, sterilization means acting on the work chamber for processing the sample and sterilizing its outer surface of the sample itself and a cooling apparatus positioned inside the isolator and employed for a sub-step of cooling the sample under a predetermined preservation temperature.

## Claims

1. A method of collection and preservation of organic fluids and/or materials containing stem cells, said fluids and/or materials comprising an amniotic liquid and/or chorionic villi, comprising the following steps:
- determining a sampling volume that is closed and/or separated from the external environment;
- taking a predetermined amount of fluid and/or material containing stem cells from said sampling volume and confiding said predetermined amount of fluid and/or material containing stem cells in a sample contained/defined in a collection volume; and
- preserving said sample;
**characterized in that**:
- said step of taking the predetermined amount of fluid and/or material is performed while continuously maintaining a perfect microbiological, atmospheric and physical isolation between the sampling volume and the collection volume; and **in that**,
- it further comprises a step of manipulating the sample under sterility conditions in an isolator apparatus, said isolator apparatus comprising:
- a work chamber isolated from an external environment and provided with a filtering system, an operator being able to accede to said work chamber by use of suitable gloves jutting out at the inside of the box and sealingly connected with one of the work chamber walls, said work chamber being pressurized to a grater pressure than the inlet chamber;
- an inlet chamber for introduction of the sample to be processed, said inlet chamber being connected to said work chamber and being provided with interlocking doors that do not allow direct communication between the work chamber and said external environment;
- an outlet chamber connected with the work chamber and having a sterile sample-collecting bag, said outlet chamber being brought into communication with the work chamber by means of interlocking doors; and
- sterilization means acting at least on the work chamber and/or the inlet chamber and/or the outlet chamber and suitable for processing the sample and sterilizing an outer surface of a container of the sample itself, a sterilization process taking place before each work step and between the operations for processing two different samples, and
- a cooling apparatus positioned inside the isolator, said cooling apparatus positioned inside the isolator being employed for a sub-step of cooling the sample under a predetermined preservation temperature.

2. A method as claimed in claim 1, wherein said predetermined amount of fluid and/or material defining the sample is comprised between 1 and 10 cc and preferably being equal to 3 cc.

3. A method as claimed in claim 1, wherein said isolator also comprises a thermo-block housed inside the work chamber and suitable for pre-cooling a freezing solution.

4. A method as claimed in claim 1, wherein said sterilization means are suitable for atomizing one or more sterilizing agents, said sterilization process taking preferably place by use of hydrogen peroxide introduced into the isolator.

5. A method as claimed in claim 1, wherein it further comprises a step of adding the fluid and/or material containing stem cells with 10% DMSO (dimethyl sulfoxide).

6. A method as claimed in claim 1, wherein it further comprises a step of continuous controlling of particle-count and/or microbiological-count parameters.

7. A method as claimed in claim 1, wherein it further comprises a step of freezing the stem cells, said step comprising the following sub-steps:
- taking an amount of amniotic liquid and/or chorionic villi, obtained through CVS (Chorionic Villi Sapling), said amount being equal to 2-2,5 ml;
- admitting said amount of amniotic liquid and/or chorionic villi into a container, said container being a 15 ml test tube with a conical bottom and a screw plug;
- inserting said test tube into the isolator;
- centrifuging said amount of an amniotic liquid and/or chorionic villi at 2000 rpm for 10 minutes; and
- taking a supernatant from said test tube.

8. A method as claimed in claim 1, wherein also present is a step of preserving the sample in suitable storage containers containing liquid nitrogen.

9. A method as claimed in claim 1, wherein it further comprises a step of defrosting the sample, said step comprising the following sub-steps:
- taking the sample from liquid nitrogen;
- positioning the sample in ice;
- bringing the sample into a thermostat at 37°C;
- transferring the sample into the isolator after said defrosting step;
- transferring the sample drop-wise into a test tube having a 15 ml capacity, conical bottom and screw plug and containing about 9 ml of a washing medium; and.
- centrifuging said test tube, after a sub-step of extracting the test tube from the isolator at 1500 rpm for 10 minutes.

10. A method as claimed in claim 9, wherein also present are the following steps:
- subsequently to the step of centrifuging a test tube, inserting said test tube into the isolator;
- taking a supernatant from said test tube;
- re-suspending a cell pellet contained in said test tube with a suitable growth substance in an amount included between 1 ml and 4 ml; and
- transferring said cell pellet and said growth substance into a flask of the "T25" type.

## Patentansprüche

1. Verfahren zum Sammeln und Konservieren von organischen Flüssigkeiten und/oder Materialien, enthaltend Stammzellen, wobei diese Flüssigkeiten und/oder Materialien eine amniotische Flüssigkeit und/oder Chorionzotten umfassen, umfassend folgende Schritte:
- Ermitteln eines Probvolumens, das geschlossen ist und/oder von der äußeren Umgebung getrennt ist;
- Entnehmen einer vorgegebenen Menge von Flüssigkeit und/oder Material, enthaltend Stammzellen, aus diesem Probvolumen, und Eingrenzen dieser vorgegebenen Menge von Flüssigkeit und/oder Material, enthaltend Stammzellen, in einer Probe, die innerhalb eines Sammelvolumens enthalten/definiert ist; und
- Konservieren dieser Probe;
**dadurch gekennzeichnet, dass**:
- dieser Schritt des Entnehmens der vorgegebenen Menge von Flüssigkeit und/oder Material ausgeführt wird, während eine perfekte mikrobiologische, atmosphärische und physische Isolierung zwischen dem Probvolumen und dem Sammelvolumen kontinuierlich gehalten wird; und dass
- er zudem einen Schritt des Manipulierens der Probe unter sterilen Bedingungen in einer Isoliervorrichtung umfasst, wobei diese Isoliervorrichtung Folgendes umfasst:
- eine Arbeitskammer, die von der äußeren Umgebung isoliert ist und mit einem Filtersystem versehen ist, wobei ein Bediener in der Lage ist, diese Arbeitskammer zu betreten, unter Verwendung von geeigneten Handschuhen, die im Innere des Kastens hervorspringen, und mit einer der Wände der Arbeitskammer abdichtend verbunden sind, wobei diese Arbeitskammer bis zu einem höheren Druck als die Eingangskammer druckbeaufschlagt ist;
- eine Eingangskammer zur Einführung der zu verarbeitenden Probe, wobei diese Eingangskammer mit der genannten Arbeitskammer verbunden ist und mit Verriegelungstüren versehen ist, die keine direkte Verbindung zwischen der Arbeitskammer und der genannten äußeren Umgebung erlauben;
- eine Ausgangskammer, die mit der Arbeitskammer verbunden ist und eine sterile Proben-Sammeltasche hat, wobei die Ausgangskammer mittels der Verriegelungstüren mit der Arbeitskammer in Verbindung gesetzt wird; und
- Sterilisationsmittel, die zumindest an der Arbeitskammer und/oder der Eingangskammer und/oder der Ausgangskammer wirken und geeignet sind, um die Probe zu verarbeiten und eine Außenfläche eines Behälters der Probe zu sterilisieren, wobei ein Sterilisationsprozess vor jedem Arbeitsschritt und zwischen den Operationen für die Verarbeitung von zwei verschiedenen Proben stattfindet, und
- eine Kühlvorrichtung, die innerhalb des Isolators positioniert ist, wobei diese innerhalb des Isolators positionierte Kühlvorrichtung verwendet wird, um einen Unterschritt des Kühlens der Probe unter einer vorgegebenen Konservierungstemperatur auszuführen.

2. Verfahren nach Anspruch 1, wobei die genannte vorgegebene Menge von Flüssigkeit und/oder Material, die die Probe definiert, zwischen 1 und 10 cm³ beträgt, und vorzugsweise gleich 3 cm³ ist.

3. Verfahren nach Anspruch 1, wobei der genannte Isolator auch einen Thermo-Block umfasst, der innerhalb der Arbeitskammer untergebracht ist und zur Vorkühlung einer Einfrierlösung geeignet ist.

4. Verfahren nach Anspruch 1, wobei die genannten Sterilisationsmittel dafür geeignet sind, eine oder mehrere Sterilisierungssubstanzen zu zerstäuben, wobei dieser Sterilisationsprozess vorzugsweise unter Verwendung von Wasserstoffperoxid, das in den Isolator eingeführt wird, stattfindet.

5. Verfahren nach Anspruch 1, zudem umfassend einen Schritt des Hinzufügens von 10% DMSO (Dimethylsulfoxid) zur Flüssigkeit und/oder zum Material, enthaltend Stammzellen.

6. Verfahren nach Anspruch 1" zudem umfassend einen Schritt des kontinuierlichen Überwachens der Partikel- und/oder mikrobiologischen Zahlparameter.

7. Verfahren nach Anspruch 1, zudem umfassend einen Schritt des Einfrierens der Stammzellen, wobei dieser Schritt die folgenden Unterschritte umfasst:
- Entnehmen einer Menge amniotischer Flüssigkeit und/oder Chorionzotten, erhaltend durch CVS (Chorionzotten-Probenahme), wobei diese Menge gleich 2-2,5 ml ist;
- Zugeben dieser Menge amniotischer Flüssigkeit und/oder Chorionzotten in einen Behälter, wobei dieser Behälter ein 15 ml Reagenzglas mit konischem Boden und einem Schraubverschluss ist;
- Einfügen dieses Reagenzglases in den Isolator;
- Zentrifugieren der genannten Menge einer amniotischen Flüssigkeit und/oder Chorionzotten bei 2000 U/Min für 10 Minuten; und
- Entnehmen eines Überstands vom genannten Reagenzglas.

8. Verfahren nach Anspruch 1, zudem aufweisend einen Schritt des Konservierens der Probe in geeigneten Lagerbehältern, die Flüssigstickstoff enthalten.

9. Verfahren nach Anspruch 1, zudem umfassend einen Schritt des Auftauens der Probe, wobei dieser Schritt die folgende Unterschritte umfasst:
- Entnehmen der Probe aus dem Flüssigstickstoff;
- Positionieren der Probe im Eis;
- Erwärmen der Probe in einem Thermostat auf 37°C;
- Übertragen der Probe in den Isolator nach dem genannten Auftauensschritt;
- tropfenweises Übertragen der Probe in ein Reagenzglas mit einem Fassungsvermögen von 15 ml, konischem Boden und Schraubverschluss und enthaltend etwa 9 ml eines Reinigungsmediums; und
- Zentrifugieren des genannten Reagenzglases, nach einem Unterschritt des Extrahierens des Reagenzglases aus dem Isolator bei 1500 U/Min für 10 Minuten.

10. Verfahren nach Anspruch 9, zudem aufweisend folgende Schritte:
- Einführen des genannten Reagenzglases in den Isolator nach dem Schritt des Zentrifugierens eines Reagenzglases;
- Entnehmen eines Überstands vom genannten Reagenzglas;
- erneutes Suspendieren eines Zellpellets, das im genannten Reagenzglas enthalten ist, mit einem geeigneten Wuchsstoff, der in einer Menge zwischen 1 ml und 4 ml enthalten ist; und
- Übertragen des genannten Zellpellets und des genannten Wuchsstoffs in einen Glaskolben des Typs "T25".

## Revendications

1. Procédé de collecte et de conservation de fluides organiques et/ou de matières contenant des cellules souches, lesdits fluides et/ou matières comprenant un liquide amniotique et/ou des villosités choriales, comportant les étapes suivantes :
- détermination d'un volume d'échantillonnage qui est fermé par rapport à l'environnement externe et/ou séparé de celui-ci ;
- prélèvement d'une quantité prédéterminée de fluide et/ou de matière contenant des cellules souches à partir dudit volume d'échantillonnage et confinement de ladite quantité prédéterminée de fluide et/ou de matière contenant des cellules souches dans un échantillon contenu/défini dans un volume de collecte ; et
- conservation dudit échantillon ;
**caractérisé en ce que** :
ladite étape de prélèvement de la quantité prédéterminée de fluide et/ou de matière est faite en même temps qu'une étape de maintien d'un parfait isolement microbiologique, atmosphérique et physique entre le volume d'échantillonnage et le volume de collecte ; et **en ce que**,
- il comprend également une étape de manipulation de l'échantillon en conditions stériles dans un appareil isolateur, ledit appareil isolateur comprenant :
- une chambre de travail isolée d'un environnement externe et équipée d'un système de filtration, un opérateur pouvant accéder à ladite chambre de travail par l'intermédiaire de gants prévus à cet effet faisant saillie vers l'intérieur du compartiment et assemblés hermétiquement à l'une des parois de la chambre de travail,
ladite chambre de travail étant soumise à une pression supérieure à celle de la chambre d'entrée ;
- une chambre d'entrée pour introduire l'échantillon à traiter, ladite chambre d'entrée étant raccordée à ladite chambre de travail et étant équipée de portes à verrouillage asservi qui empêchent toute communication directe entre la chambre de travail et ledit environnement externe ;
- une chambre de sortie raccordée à la chambre de travail et ayant un sachet stérile d'échantillonnage, ladite chambre de sortie étant mise en communication avec la chambre de travail par le biais des portes à verrouillage asservi ; et
- des moyens de stérilisation agissant au moins sur la chambre de travail et/ou la chambre d'entrée et/ou la chambre de sortie, et en mesure de traiter l'échantillon et de stériliser une surface externe d'un récipient de l'échantillon, un procédé de stérilisation se produisant avant chaque étape de travail et entre les opérations de traitement de deux différents échantillons, et
- un appareil de refroidissement positionné à l'intérieur de l'isolateur, ledit appareil de refroidissement positionné à l'intérieur de l'isolateur servant à une sous-étape de refroidissement de l'échantillon à une température de conservation prédéterminée.

2. Procédé selon la revendication 1, dans lequel ladite quantité prédéterminée de fluide et/ou de matière formant l'échantillon est comprise entre 1 et 10 cm³, et de préférence est égale à 3 cm³.

3. Procédé selon la revendication 1, dans lequel ledit isolateur comprend également un thermobloc placé à l'intérieur de la chambre de travail et pouvant pré-refroidir une solution réfrigérante.

4. Procédé selon la revendication 1, dans lequel lesdits moyens de stérilisation sont en mesure d'atomiser un ou plusieurs agents de stérilisation, ledit procédé de stérilisation se déroulant de préférence en introduisant du peroxyde d'hydrogène dans l'isolateur.

5. Procédé selon la revendication 1, dans lequel est également comprise une étape d'ajouter 10% DMSO (diméthylsulfoxyde) au fluide et/ou à la matière contenant des cellules souches.

6. Procédé selon la revendication 1, dans lequel est aussi comprise une étape de contrôler en continu les paramètres de comptage des particules et/ou de comptage microbiologique.

7. Procédé selon la revendication 1, dans lequel est aussi comprise une étape de réfrigérer les cellules souches, ladite étape comprenant les sous-étapes suivantes :
- prélèvement d'une quantité de liquide amniotique et/ou de villosités choriales, par PVC (prélèvement des villosités choriales), ladite quantité étant égale à 2-2,5 ml ;
- injection de ladite quantité de liquide amniotique et/ou des villosités choriales dans un récipient, ledit récipient étant une éprouvette de 15 ml avec un fond conique et un bouchon fileté ;
- insertion de ladite éprouvette dans l'isolateur ;
- centrifugation de ladite quantité de liquide amniotique et/ou des villosités choriales à 2 000 tours/minute pendant 10 minutes ; et
- prélèvement d'un surnageant dans ladite éprouvette.

8. Procédé selon la revendication 1, dans lequel est également comprise une étape de conservation de l'échantillon dans des récipients de conservation appropriés contenant de l'azote liquide.

9. Procédé selon la revendication 1, dans lequel est aussi comprise une étape de dégivrer l'échantillon, ladite étape comprenant les sous-étapes suivantes :
- retrait de l'échantillon de l'azote liquide ;
- positionnement de l'échantillon dans la glace ;
- placement de l'échantillon dans un thermostat à 37 °C ;
- transfert de l'échantillon dans l'isolateur après ladite étape de dégivrage ;
- transfert de l'échantillon goutte à goutte dans une éprouvette d'une capacité de 15 ml avec fond conique et bouchon fileté et contenant environ 9 ml de milieu de lavage ; et - centrifugation de ladite éprouvette, après une sous-étape d'extraction de l'éprouvette de l'isolateur à 1 500 tours/minute pendant 10 minutes.

10. Procédé selon la revendication 9, dans lequel les étapes suivantes sont également présentes :
- après l'étape de centrifugation d'une éprouvette, insertion de ladite éprouvette dans l'isolateur ;
- prélèvement d'un surnageant dans ladite éprouvette ;
- remise en suspension d'un culot de cellules contenu dans ladite éprouvette avec une substance de croissance appropriée dans une quantité comprise entre 1 ml et 4 ml ; et
- transfert dudit culot de cellules et de ladite substance de croissance dans une flasque de type « T25 ».
